# EUROPEAN PATENT SPECIFICATION

(11) **EP 2 691 097 B1**
(45) Date of publication and mention of the grant of the patent: **20.12.2017**
(21) Application number: 11720901.5
(22) Date of filing: 31.03.2011
(51) Int. Cl.: A61K 31/485, A61P 43/00, A61K 9/48, A61K 9/06, A61K 9/00

(54) **COMPOSITIONS COMPRISING NALTREXONE FOR USE IN TREATING SCLERODERMA**
ZUBEREITUNGEN ENTHALTEND NALTREXON ZUR VERWENDUNG BEI DER BEHANDLUNG VON SKLERODERMIE
COMPOSITIONS COMPRENANT DE LA NALTREXONE POUR UTILISATION DANS LE TRAITEMENT DE LA SCLÉRODERMIE

(43) Date of publication of application: 05.02.2014
(73) Proprietor: Imuneks Farma Ilaç Sanayi Ve Ticaret A.S., 34349 Istanbul (TR)
(72) Inventor: PISAK, Ibrahim Mustafa Iskender, Gayrettepe / Istanbul 34349 (TR); PISAK, Mehmet, Gayrettepe / Istanbul 34349 (TR); SELAMOGLU, Mehmet Levent, Gayrettepe / Istanbul 34349 (TR); BINGOL, Semra, Gayrettepe / Istanbul 34349 (TR)
(74) Representative: Kodron, Felix
(86) International application number: PCT/TR2011/000075
(87) International publication number: WO 2012/134410

(56) References cited:
- WO-A2-2007/121447
- DATABASE BIOSIS [Online] BIOSCIENCES INFORMATION SERVICE, PHILADELPHIA, PA, US; 1984, WILES P G ET AL: "THE EFFECT OF NALOXONE ON SKIN BLOOD FLOW IN NORMAL PERSONS AND PATIENTS WITH RAYNAUDS DISEASE", XP009145988, Database accession no. PREV198528090474 & QJM, vol. 53, no. 212, 1984, pages 530-531, 78TH ANNUAL GENERAL MEETING OF THE ASSOCIATION OF PHYSICIANS OF GREAT BRITAIN AND IRELAND, LEICESTER ISSN: 0033-5622
- STREHLOW D ET AL: "Biology of the scleroderma fibroblast", CURRENT OPINION IN RHEUMATOLOGY, CURRENT SCIENCE, LONDON, GB, vol. 10, no. 6, 1 November 1998 (1998-11-01), pages 572-578, XP009154918, ISSN: 1040-8711 cited in the application
- EBRAHIMKHANI M R ET AL: "Naltrexone, an opioid receptor antagonist, attenuates liver fibrosis in bile duct ligated rats", GUT, BRITISH MEDICAL ASSOCIATION, LONDON, UK, vol. 55, no. 11, 1 November 2006 (2006-11-01), pages 1606-1616, XP009154888, ISSN: 0017-5749
- D'ANGELO W A ET AL: "Pathologic observations in systemic sclerosis (scleroderma)", AMERICAN JOURNAL OF MEDICINE, EXCERPTA MEDICA, INC, UNITED STATES, vol. 46, no. 3, 1 March 1969 (1969-03-01), pages 428-440, XP026357292, ISSN: 0002-9343, DOI: 10.1016/0002-9343(69)90044-8 [retrieved on 1969-03-01]
- RAU R ET AL: "Zur Frage einer Leberbetelligung bei progressiver Sklerodermie", SCHWEIZERISCHE MEDIZINISCHE WOCHENSCHRIFT, E M H SCHWEIZERISCHER AERZTEVERLAG AG, CH, vol. 104, no. 50, 1 January 1974 (1974-01-01), pages 1877-1879, XP009154917, ISSN: 0036-7672

## Description

### Field of the Invention

The invention relates to compositions comprising opioid antagonists, such as naltrexone, and the use of said pharmaceutical compositions for treating scleroderma, especially systemic sclerosis.

### Background of the Invention

Naltrexone has the chemical name of morphinan-6-one, 17-(cyclopropylmethyl)-4,5-epoxy-3,14-dihydroxy-(5α). The molecular formula of naltrexone is C₂₀H₂₃NO₄ and its molecular weight is 341.41 in the anhydrous form (< 1% maximum water content). The chemical structure of naltrexone is shown below.

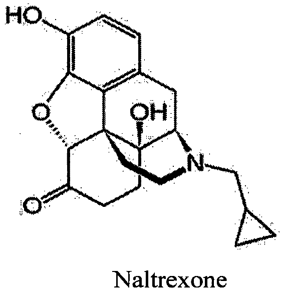

Naltrexone has been approved for use in the treatment of alcoholism or narcotic addiction. It is believed that naltrexone functions by blocking the brain receptors that trigger the effects of alcohol or narcotics. Naltrexone is marketed by Durmed in the form of a tablet under the tradename ReVia® and by Alkermes in the form of a powder for injectable suspension under the tradename Vivitrol®.

Naloxone has the chemical name of (-)-17-allyl-4,5α-epoxy-3,14-dihydroxymorphinan-6-one and the molecular formula of C₁₉H₂₁NO₄. The chemical structure of naloxone is shown below.

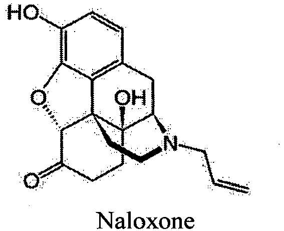

Naloxone is typically administered intravenously because of its short duration of action, and is generally administered to a patient in order to reverse opioid depression, including respiratory depression, induced by natural and synthetic opioids. The effect of naloxone on skin blood flow in normal persons and patients with Raynauds disease is discussed in an article of Wiles P.G. et al., Database Biosis (online) Bioscience information service, Philadelphia, PA, US PREV 198528090474, QJM, vol 53, no. 212, 1984, pages 530-531.

Nalmefene has the chemical name of 17-cyclopropylmethyl-4,5α-epoxy-6-methylenemorphinan-3,14-diol and the molecular formula of C₂₁H₂₅NO₃. The chemical structure of nalmefene is shown below.

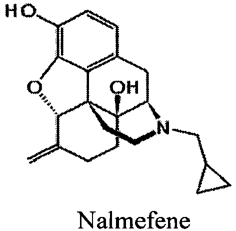

Nalmefene is typically used in the management of alcohol dependence, and also has been investigated for the treatment of other addictions such as pathological gambling and addiction to shopping. Advantages of nalmefene relative to naltrexone include longer half-life, greater oral bioavailability and no observed dose-dependent liver toxicity. As with other drugs of this type, nalmefene can precipitate acute withdrawal symptoms in patients who are dependent on opioid drugs, or more rarely when used post-operatively to counteract the effects of strong opioids used in surgery.

In the early 1980s, it was reported that the administration of low dose naltrexone (less than 10 mg naltrexone per day) increases the production of endogenous endomorphins, especially the endogenous pentapeptide metenkephalin, and increases the number and density of metenkephalin receptors by intermittently blocking opiate receptors (I.S. Zagon & P.J. McLaughlin, "Naltrexone modulates tumor response in mice with neuroblastoma", Science, 221: 671-3 (12 Aug 1983). This increase in metenkephalin is believed to enhance homeostatic regulation of the natural immune function of the human body.

In view of Zagon's findings, Bernard Bihari reported the use of low dose naltrexone for the treatment of patients with AIDS (U.S. Patent No. 4,888,346) and herpes (U.S. Patent No. 5,356,900). Further, Nicholas Plotnikoff reported the use of low dose naltrexone for the treatment of herpes, HIV infection, cytomegalovirus, coronavirus, influenza A and Japanese encephalitis. (E.A. Moore & S. Wilkinson, THE PROMISE OF Low DOSE NALTREXONE THERAPY: POTENTIAL BENEFITS IN CANCER, AUTOIMMUNE, NEUROLOGICAL AND INFECTIOUS DISORDERS (McFarland & Company, Inc., Publishers, 2009)).

Scleroderma is a rare disease that affects approximately 19 out of every 1 million people. The cause of scleroderma is unknown, but it is believed that there may be a genetic predisposition for the disease. Genetic abnormalities that have been observed in people diagnosed with scleroderma involve autoimmunity and alteration of endothelial cell and fibroblast function. Indeed, systemic sclerosis is probably the most severe of the autoimmune diseases with 50% mortality within 5 years of diagnosis (Silman A. J. (1991) Mortality from scleroderma in England and Wales 1968-1975. Ann. Rheu. Dis. 50, 95-96).

Scleroderma has a spectrum of manifestations and a variety of therapeutic implications. Types of scleroderma include localized scleroderma, systemic sclerosis, scleroderma-like disorders, and Sine scleroderma (Smith, Textbook of the Autoimmune Diseases, Edited by Lahita, Chiorazzi and Reeves, Lippincott Williams & Wilkins, Philadelphia 2000).

While localized scleroderma is a rare dermatologic disease associated with fibrosis and manifestations limited to skin, systemic sclerosis is a multisystem disease with variable risk for internal organ involvement and variation in the extent of skin disease. Systemic sclerosis can be diffuse or limited. Limited systemic sclerosis is also called CREST (calcinosis, Raynaud's esophageal dysfunction, sclerodactyly, telangiectasiae). Scleroderma-like disorders are believed to be related to industrial environment exposure. In Sine disease, there is internal organ involvement without skin changes.

The major manifestations of scleroderma and in particular of systemic sclerosis are inappropriate excessive collagen synthesis and deposition, endothelial dysfunction, spasm, collapse and obliteration by fibrosis. Several underlying biological processes are implicated in the initiation, severity and progression of the disease and include vascular dysfunction, endothelial cell activation and damage, leukocyte accumulation, auto-antibody production and crucially, an uncontrolled fibrotic response which may lead to death (Clements P. J. and Furst D. E. (1996) "Systemic Sclerosis" Williams and Williams, Baltimore).

Fibroblasts have a pivotal role in the pathogenesis of this disease. Primary fibroblasts obtained from patients with scleroderma exhibit many of the characteristic properties of the disease seen in vivo, notably increased extracellular matrix synthesis and deposition, notably of collagen and fibronectin, and altered growth factor and cytokine production such as of transforming growth factor (TGF) and connective tissue growth factor (CTGF) (Strehlow D. and Korn J (1998) Biology of the scleroderma fibroblast. Curr. Opin. Rheumatol. 10, 572-578; LeRoy E. C. (1974) Increased collagen synthesis by scleroderma skin fibroblasts in vitro. J. Clin. Invest. 54, 880-889). There is no curative treatment of scleroderma. Innovative but high-risk therapy proposed autologous stem cell transplantation (Martini, Maccado, Ravelli et al., Arthritis Rheum. 1999, 42, 807-811).

### Summary of the Invention

The invention relates to naltrexone for use in the treatment of scleroderma comprising administering to a subject in need thereof an effective amount of the opioid antagonist.

### Detailed Description of the Invention

The present invention relates to the use for treating scleroderma comprising administering to a subject in need thereof an effective amount of naltrexone or a composition comprising naltrexone and at least one pharmaceutically acceptable excipient for the treatment of scleroderma.

As used herein, an "effective amount" is an amount effective for treating scleroderma.

As used herein, the term "treating" scleroderma in a subject refers to subjecting the subject to a pharmaceutical treatment, e.g., the administration of one or more agents, such that at least one symptom of the condition is decreased or prevented from worsening.

In one embodiment, the scleroderma is localized scleroderma, systemic sclerosis, a scleroderma-like disorder. The symptoms of scleroderma to be treated include inappropriate excessive collagen synthesis and deposition, endothelial dysfunction, spasm, collapse and obliteration by fibrosis.

Suitable opioid antagonists include compounds that block one or more opioid receptors. It is claimed in claim 1 that the opioid antagonist is naltrexone.

The opioid antagonist is typically administered to the subject in the form of a composition for topical or oral administration (claims 4 and 12). In another embodiment, disclosed in claim 2, the composition comprises an effective amount of the opioid antagonist naltrexone and at least one pharmaceutically acceptable excipient.

### Compositions for topical administration

In claim 4 it is defined, that the opioid antagonist is administered to the subject in the form of a composition for topical administration.

In another embodiment, the opioid antagonist is present in the topical compositions of the invention in an amount of from about 0.1% to about 5% by weight of the composition (claim 5). In another embodiment, the opioid antagonist is present in an amount of from about 0.5% to about 2% (claim 6), or about 1% by weight of the composition (claim 7).

Suitable pharmaceutically acceptable excipient for the topical compositions of the invention may be any topically acceptable non-transdermally effective excipient known by those skilled in the art. Suitable excipients include, but are not limited to, emulsifying agents, stiffening agents, rheology modifiers or thickeners, surfactants, emollients, preservatives, humectants, alkalizing or buffering agents, and solvents.

Suitable emulsifying agents include, but are not limited to, cetyl alcohol, cetostearyl alcohol, stearyl alcohol, carboxypolymethylene, polycarbophil, polyethylene glycol, and sorbitan esters. Suitable stiffening agents include, but are not limited to, stearyl alcohol, cetostearyl alcohol, and cetyl alcohol. Suitable rheology modifiers or thickeners include, but are not limited to, carbomers such as Carbopol®, and polyoxyethylene tallow amines such as Ethomeen®. Suitable surfactants include, but are not limited to, anionic, cationic, amphoteric, and nonionic surfactants. In some embodiments, the surfactant is sodium lauryl sulfate, cetostearyl alcohol, cetyl alcohol, magnesium lauryl sulfate, a wax, or a combination thereof. Suitable emollients include, but are not limited to, white petrolatum (while vaseline), liquid petrolatum (liquid vaseline), paraffin, or aquaphor. Suitable preservatives include, but are not limited to, antimicrobial preservatives such as nipagin (methyl hydroxybenzoate), nipasol (hydroxybenzoate), butylparaben, ethylparaben, methylparaben, propyl paraben potassium, and propyl paraben sodium. Suitable humectants include, but are not limited to, propylene glycol and propylene glycol alginate. Suitable alkalizing or buffering agents include, but are not limited to, sodium hydroxide and potassium hydroxide. Suitable solvents include, but are not limited to, water.

The composition may be in the form of a gel, cream, ointment, liquid, suspension, solution, emulsion, foam, aerosol or the like for topical administration (claim 8). Typically, the composition is administered to the subject by spreading (*e.g.*, gel, cream, or ointment) or spraying (*e.g.*, liquid) onto the affected area of the skin.

In one embodiment, the composition is in the form of cream. Typically, the cream comprises an opioid antagonist and one or more of an emulsifying agent, a stiffening agent, a surfactant, an emollient, a preservative, a humectant, an alkalizing or buffering agent, and a solvent. The cream has a composition according to Table 1a, 1b, 1c.

**Table 1a. Illustrative Cream Composition**

| **Ingredient** | **Amount (% by weight of the composition)** |
|---|---|
| Opioid antagonist | about 0.1% - 5% |
| Emulsifying agent | about 2% - 5% |
| Stiffening agent | about 1% - 45% |
| Surfactant | about 0.5% - 2.5% |
| Preservative | about 0.01% - 0.6% |
| Humectant | about 1% - 15% |
| Alkalizing or buffering agent | about 0.01% - 3% |
| Emollient | about 1% - 50% |
| Solvent | q.s. |

**Table 1b. Illustrative Cream Composition**

| **Ingredient** | **Amount (% by weight of the composition)** |
|---|---|
| Opioid antagonist | about 0.5% - 2% |
| Emulsifying agent | about 2% - 5% |
| Stiffening agent | about 2% - 5% |
| Surfactant | about 0.5% - 1.5% |
| Preservative | about 0.01% - 0.6% |
| Humectant | about 2% - 10% |
| Alkalizing or buffering agent | about 0.01% - 3% |
| Emollient | about 15% - 30% |
| Solvent | q.s. |

**Table 1c. Illustrative Cream Composition**

| **Ingredient** | **Amount (% by weight of the composition)** |
|---|---|
| Opioid antagonist | about 0.5% - 2% |
| Cetyl alcohol and/or carboxypolymethylene | about 2% - 5% |
| Stearyl alcohol | about 2% - 5% |
| Sodium lauryl sulfate | about 0.5% - 1.5% |
| Nipagin and/or Nipasol | about 0.01% - 0.6% |
| Propylene glycol | about 2% - 10% |
| Sodium hydroxide | about 0.01% - 3% |
| White vaseline and/or liquid vaseline | about 15% - 30% |
| Water | q.s. |

In one embodiment, the composition is in the form of ointment. Typically, the ointment comprises an opioid antagonist and one or more of an emulsifying agent, an emollient, and a preservative. The ointment has a composition according to Table 2a, 2b, 2c.

**Table 2a. Illustrative Ointment Composition**

| **Ingredient** | **Amount (% by weight of the composition)** |
|---|---|
| Opioid antagonist | about 0.1% - 5% |
| Emulsifying agent | about 1% - 10% |
| Preservative | about 0.01% - 0.6% |
| Emollient | q.s. |

**Table 2b. Illustrative Ointment Composition**

| **Ingredient** | **Amount (% by weight of the composition)** |
|---|---|
| Opioid antagonist | about 0.5% - 2% |
| Emulsifying agent | about 2% - 5% |
| Preservative | about 0.01% - 0.6% |
| Emollient | q.s. |

**Table 2c. Illustrative Ointment Composition**

| **Ingredient** | **Amount (% by weight of the composition)** |
|---|---|
| Opioid antagonist | about 0.5% - 2% |
| Polyoxyethylene 20 sorbitan monooleate | about 2% - 5% |
| Nipagin and/or Nipasol | about 0.01 % - 0.6% |
| White vaseline and/or liquid vaseline | q.s. |

In one embodiment, the composition is in the form of gel. Typically, the gel comprises an opioid antagonist and one or more of a rheology modifier or thickener, an alkalizing or buffering agent, and a solvent. The gel has a composition according to Table 3a, 3b, 3c.

**Table 3a. Illustrative Gel Composition**

| **Ingredient** | **Amount (% by weight of the composition)** |
|---|---|
| Opioid antagonist | about 0.1% - 5% |
| Rheology modifier or thickener | about 0.5% - 2% |
| Alkalizing or buffering agent | about 0.5% - 10% |
| Solvent | q.s. |

**Table 3b. Illustrative Gel Composition**

| **Ingredient** | **Amount (% by weight of the composition)** |
|---|---|
| Opioid antagonist | about 0.5% - 2% |
| Rheology modifier or thickener | about 1% - 2% |
| Alkalizing or buffering agent | about 0.5% - 5% |
| Solvent | q.s. |

**Table 3c. Illustrative Gel Composition**

| **Ingredient** | **Amount (% by weight of the composition)** |
|---|---|
| Opioid antagonist | about 0.5% - 2% |
| Carbomer | about 1% - 2% |
| Sodium hydroxide | about 0.5% - 5% |
| Water | q.s. |

Typically, the opioid antagonist is administered to the subject in a total daily dose of up to about 150 mg/cm² of skin (disclosed in claim 9). In some embodiment (claim 10), the opioid antagonist is administered to the subject in a total daily dose of from about 10 mg/cm² of skin to about 100 mg/cm² of skin, from about 20 mg/cm² of skin to about 90 mg/cm² of skin, from about 30 mg/cm² of skin to about 80 mg/cm² of skin, from about 40 mg/cm² of skin to about 70 mg/cm² of skin, or about 50 mg/cm² of skin or about 60 mg/cm² of skin. The total daily dose may be delivered once per day, or divided between multiple doses. In some embodiments, the opioid antagonist is administered 1, 2, 3, 4, or 5 times per day (claim 17).

### Compositions for oral administration

In some embodiment (claim 12), the opioid antagonist is administered to the subject in the form of a composition for oral administration.

In some embodiments (Claims 13-15), the opioid antagonist is present in the oral compositions of the invention in an amount of from about 0.1 mg to about 10 mg, from about 1 mg to about 4.5 mg, or about 2 mg.

Suitable pharmaceutically acceptable excipients for the solid oral compositions of the invention include, but are not limited to, starches, sugars, diluents, granulating agents, lubricants, binders, disintegrating agents and the like. Suitable pharmaceutically acceptable excipients for the liquid oral compositions of the invention include, but are not limited to, water, glycols, oils, alcohols, flavoring agents, preservatives, coloring agents and the like.

The oral composition may be in the form of a tablet, capsule, caplet, granule, powder, lozenge, troche, dragee, sachet, cachet, liquid, solution, suspension, emulsion, elixir, or syrup for oral administration. In one embodiment, the oral composition is in the form of a tablet or capsule (claim 16). The tablet may be in the form of an uncoated tablet, coated tablet (for example with sugar or an enteric coating), effervescent tablet, dispersible tablet, orally- dissolving tablet, or sublingual tablet.

The topical and oral compositions described above may be administered to the subject daily, every other day, three times a week, twice a week, once a week, or at other appropriate intervals. It is claimed in claim 17, that the composition is administered in 1 to 5 times daily.

The composition is administered until there is complete healing of the scleroderma in the affected area.

The present invention may use lower doses of opioid antagonist than the doses conventionally used for oral administration in the treatment of alcohol or narcotic addiction. The opioid antagonist is administered to the individual in an amount effective to treat the scleroderma. In certain embodiments, the exact dose of opioid antagonist depends upon, by way of non-limiting example, the form in which the opioid antagonist is administered, the subject to be treated, the age, body weight and/or height of the subject to be treated, the preference and experience of the attending physician, the specific opioid antagonist used, the characteristics of the patient, and/or the nature of the condition for which the treatment is sought. Thus, in some embodiments, the dosage of opioid antagonist administered may vary from those disclosed herein. In various embodiments, these factors are determined by those of skill in the medical and pharmaceutical arts in view of the present disclosure.

The use of the invention may further comprise administration of one or more additional agents effective to treat the scleroderma (claim 18). The additional agent may include, but not be limited to, interferon, in particular interferon-α, a Tumor Necrosis Factor (TNF) antagonist, in particular TNF binding protein I and/or II (TBP I and/or TBP II), or an anti-scleroderma agent selected from the group consisting of angiotensin-converting enzyme (ACE) inhibitors, calcium channel blockers, proton pump inhibitors, non- steroidal anti-inflammatory agents (NSAIDs), cyclooxygenase (COX) inhibitors, corticosteroids, tetracycline, pentoxifylline, bucillamine, geranylgeranyl transferase inhibitors, rotterlin, prolyl-4-hydroxlase inhibitors, c-proteinase inhibitors, lysyl-oxidase inhibitors, relaxin, halofuginone, prostaglandins, prostacyclins, endothelin-1, nitric oxide, angiotensin II inhibitors and anti-oxidants. The additional agent and the opioid antagonist may be administered concurrently or separately. When administered concurrently, the additional agent and the opioid antagonist may be administered in the same or separate compositions.

Disclosed but not claimed are kits comprising a unit dose of opioid antagonist. In one embodiment the unit dose is within a container, which can be sterile, containing an effective amount of opioid antagonist and a pharmaceutically acceptable excipient. The kits can further comprise a label or printed instructions instructing the use of the opioid antagonist to treat scleroderma. The kits can further comprise a device that is useful for administering the unit dose as described herein. Examples of such a device include, but are not limited to, a wand, a dropper, a cotton swab, a pad, or the like.

Having described the invention with reference to certain embodiments, other embodiments will become apparent to one skilled in the art from consideration of the specification. The invention is further defined by reference to the following examples. It will be apparent to those skilled in the art that many modifications, both to materials and methods, may be practiced without departing from the scope of the invention.

### Examples

### Example 1: Treatment of Systemic Sclerosis with Naltrexone

### Topical composition of naltrexone

100 g of naltrexone 1% cream was prepared by mixing 1 g of naltrexone hydrochloride in 99 g of a cream base containing the following excipients: cetyl alcohol, stearyl alcohol, sodium lauryl sulfate, vaseline, nipagin, nipasol, carboxypolymethylene, propyleneglycol, sodium hydroxide and distilled water.

### Oral composition of naltrexone

A man in his 60s with manifestations of systemic sclerosis including inappropriate excessive collagen synthesis and deposition, endothelial dysfunction, spasms and fibrosis, was treated with the 1% naltrexone cream and 2 mg naltrexone capsules.

The naltrexone cream was administered to the affected area of skin three times daily (morning, noon, and evening). The hardening of the skin mostly on the hands, likely caused by collagen synthesis and fibrosis was prevented and became less and less bothersome to the subject, after the use of the cream for 1 month on the hands.

At the same time the cream was being administered, the patient was also administered the naltrexone capsule once daily before bedtime to treat the remaining symptoms. After 3 weeks of treatment, the patient started to breathe much better, most likely as a result of the minimization of the fibrosis in the lungs.

### Example 2: Naltrexone Oral and Topical Compositions

The following are illustrative naltrexone compositions according to the present invention.

**Table 4. Illustrative Naltrexone Capsule Composition**

| **Ingredient** | **Amount (mg)** |
|---|---|
| Naltrexone hydrochloride | 2.00 mg |
| Magnesium stearate | 0.18 mg |
| Microcristalline cellulose | 90.00 mg |
| Gelatin capsule No 4 | 1 |

**Table 5. Illustrative Naltrexone Cream Composition**

| **Ingredient** | **Amount (% by weight of the composition)** |
|---|---|
| Naltrexone | 1% |
| Cetyl alcohol | 3.6% |
| Stearyl alcohol | 3.6% |
| Sodium lauryl sulfate | 0.8% |
| Nipagin | 0.1% |
| Nipasol | 0.05% |
| Carboxylpolymethylene | 0.2% |
| Propylene glycol | 5% |
| Sodium hydroxide | 0.03% |
| White vaseline | 13.5% |
| Liquid vaseline | 5.4% |
| Water | q.s. |

**Table 6. Illustrative Naltrexone Ointment Composition**

| **Ingredient** | **Amount (% by weight of the composition)** |
|---|---|
| Naltrexone | 1% |
| Polyoxyethylene 20 sorbitan monooleate | 4% |
| Nipagin | 0.18% |
| Nipasol | 0.02% |
| White vaseline | 10% |
| Liquid vaseline | q.s. |

**Table 7. Illustrative Naltrexone Gel Composition**

| **Ingredient** | **Amount (% by weight of the composition)** |
|---|---|
| Naltrexone | 1% |
| Carbomer | 2% |
| Sodium hydroxide | 1.25% |
| Water | q.s. |

## Claims

1. Naltrexone for use in the treatment of scleroderma.

2. A composition comprising naltrexone and at least one pharmaceutically acceptable excipient for use in the treatment of scleroderma.

3. Naltrexone for use according to claims 1 or 2, wherein the scleroderma is systemic sclerosis. A composition for use according to claim 2, wherein the composition is formulated for topical

4. administration.

5. A composition for use according to claim 4, wherein naltrexone is present in an amount of from 0.1% to 5% by weight of the composition.

6. A composition for use according to claim 4, wherein naltrexone is present in an amount of from 0.5% to 2% by weight of the composition.

7. A composition for use according to claim 4, wherein naltrexone is present in an amount of 1% by weight of the composition.

8. A composition for use according to any one of claims 4 to 7, wherein the composition is in the form of a gel, cream, ointment, liquid, suspension, solution, emulsion, foam, or aerosol administered to the subject by spreading or spraying the composition onto the affected area.

9. A composition for use according to any one of claims 4 to 8, wherein the composition is administered in a total daily dose of the opioid antagonist up to 150 mg/cm² of skin.

10. A composition for use according to any one of claims 4 to 8, wherein the composition is administered in a total daily dose of the opioid antagonist between 10 mg/cm² and 100 mg/cm² of skin. A composition for use according to any one of claims 4 to 8, wherein the composition is

11. administered in a total daily dose of the opioid antagonist of 50 mg/cm² of skin.

12. A composition for use according to claim 2, wherein the composition is formulated for oral administration.

13. A composition for use according to claim 12, wherein naltrexone is present in an amount of from 0.1 mg to 10 mg.

14. A composition for use according to claim 12, wherein naltrexone is present in an amount of from 1 mg to 4.5 mg.

15. A composition for use according to claim 12, wherein naltrexone is present in an amount of 2 mg. A composition for use according to any one of claims 12 to 15, wherein the composition is in the

16. form of a tablet or capsule.

17. A composition for use according to any one of claims 2 to 16, wherein the composition is administered 1 to 5 times daily.

18. A composition for use according to any one of claims 2 to 17, wherein the composition further comprises administering to the subject one or more additional agents effective to treat scleroderma.

## Patentansprüche

1. Naltrexon zur Verwendung bei der Behandlung von Sklerodermie.

2. Zusammensetzung, die Naltrexon und mindestens einen pharmazeutisch unbedenklichen Hilfsstoff umfasst, zur Verwendung bei der Behandlung von Sklerodermie.

3. Naltrexon zur Verwendung nach Anspruch 1 oder 2, wobei die Sklerodermie systemische Sklerose ist.

4. Zusammensetzung zur Verwendung nach Anspruch 2, wobei die Zusammensetzung zur topischen Verabreichung formuliert ist.

5. Zusammensetzung zur Verwendung nach Anspruch 4, wobei Naltrexon in einer Menge von 0,1 Gewichts-% bis 5 Gewichts-% der Zusammensetzung vorliegt.

6. Zusammensetzung zur Verwendung nach Anspruch 4, wobei Naltrexon in einer Menge von 0,5 Gewichts-% bis 2 Gewichts-% der Zusammensetzung vorliegt.

7. Zusammensetzung zur Verwendung nach Anspruch 4, wobei Naltrexon in einer Menge von 1 Gewichts-% der Zusammensetzung vorliegt.

8. Zusammensetzung zur Verwendung nach einem der Ansprüche 4 bis 7, wobei die Zusammensetzung in Form eines Gels, einer Creme, einer Salbe, einer Flüssigkeit, einer Suspension, einer Lösung, einer Emulsion, eines Schaums oder eines Aerosols vorliegt, das bzw. die bzw. der der Person durch Verstreichen oder Aufsprühen der Zusammensetzung auf dem betroffenen Bereich verabreicht wird.

9. Zusammensetzung zur Verwendung nach einem der Ansprüche 4 bis 8, wobei die Zusammensetzung in einer täglichen Gesamtdosis des Opioid-Antagonisten von bis zu 150 mg/cm² Haut verabreicht wird.

10. Zusammensetzung zur Verwendung nach einem der Ansprüche 4 bis 8, wobei die Zusammensetzung in einer täglichen Gesamtdosis des Opioid-Antagonisten von 10 mg/cm² bis 100 mg/cm² Haut verabreicht wird.

11. Zusammensetzung zur Verwendung nach einem der Ansprüche 4 bis 8, wobei die Zusammensetzung in einer täglichen Gesamtdosis des Opioid-Antagonisten von 50 mg/cm² Haut verabreicht wird.

12. Zusammensetzung zur Verwendung nach Anspruch 2, wobei die Zusammensetzung zur oralen Verabreichung formuliert ist.

13. Zusammensetzung zur Verwendung nach Anspruch 12, wobei Naltrexon in einer Menge von 0,1 mg bis 10 mg vorliegt.

14. Zusammensetzung zur Verwendung nach Anspruch 12, wobei Naltrexon in einer Menge von 1 mg bis 4,5 mg vorliegt.

15. Zusammensetzung zur Verwendung nach Anspruch 12, wobei Naltrexon in einer Menge von 2 mg vorliegt.

16. Zusammensetzung zur Verwendung nach einem der Ansprüche 12 bis 15, wobei die Zusammensetzung in Form einer Tablette oder Kapsel vorliegt.

17. Zusammensetzung zur Verwendung nach einem der Ansprüche 2 bis 16, wobei die Zusammensetzung 1 bis 5 Mal täglich verabreicht wird.

18. Zusammensetzung zur Verwendung nach einem der Ansprüche 2 bis 17, wobei die Zusammensetzung ferner die Verabreichung von einem oder mehreren zusätzlichen Mitteln, die zur Behandlung von Sklerodermie wirksam sind, an die Person umfasst.

## Revendications

1. Naltrexone pour utilisation dans le traitement de la sclérodermie.

2. Composition comprenant de la naltrexone et au moins un excipient pharmaceutiquement acceptable pour utilisation dans le traitement de la sclérodermie.

3. Naltrexone pour utilisation selon les revendications 1 ou 2, où la sclérodermie est la sclérose systémique.

4. Composition pour utilisation selon la revendication 2, où la composition est formulée pour administration topique.

5. Composition pour utilisation selon la revendication 4, où la naltrexone est présente à une teneur comprise entre 0,1 % et 5 % en masse de la composition.

6. Composition pour utilisation selon la revendication 4, où la naltrexone est présente à une teneur comprise entre 0,5 % et 2 % en masse de la composition.

7. Composition pour utilisation selon la revendication 4, où la naltrexone est présente à une teneur de 1 % en masse de la composition.

8. Composition pour utilisation selon l'une quelconque des revendications 4 à 7, où la composition se présente sous la forme d'un gel, d'une crème, d'un onguent, d'un liquide, d'une suspension, d'une solution, d'une émulsion, d'une mousse ou d'un aérosol administrés au sujet par étalement ou vaporisation de la composition sur la zone affectée.

9. Composition pour utilisation selon l'une quelconque des revendications 4 à 8, où la composition est administrée à une dose quotidienne totale de l'antagoniste d'opioïde allant jusqu'à 150 mg/cm² de peau.

10. Composition pour utilisation selon l'une quelconque des revendications 4 à 8, où la composition est administrée à une dose quotidienne totale de l'antagoniste d'opioïde comprise entre 10 mg/cm² et 100 mg/cm² de peau.

11. Composition pour utilisation selon l'une quelconque des revendications 4 à 8, où la composition est administrée à une dose quotidienne totale de l'antagoniste d'opioïdes de 50 mg/cm² de peau.

12. Composition pour utilisation selon la revendication 2, où la composition est formulée pour administration orale.

13. Composition pour utilisation selon la revendication 12, où la naltrexone est présente en une quantité de 0,1 mg à 10 mg.

14. Composition pour utilisation selon la revendication 12, où la naltrexone est présente en une quantité de 1 mg à 4,5 mg.

15. Composition pour utilisation selon la revendication 12, où la naltrexone est présente en une quantité de 2 mg.

16. Composition pour utilisation selon l'une quelconque des revendications 12 à 15, où la composition se présente sous la forme d'un comprimé ou d'une capsule.

17. Composition pour utilisation selon l'une quelconque des revendications 2 à 16, où la composition est administrée 1 à 5 fois par jour.

18. Composition pour utilisation selon l'une quelconque des revendications 2 à 17, où la composition comprend en outre l'administration au sujet d'un ou de plusieurs agents supplémentaires efficaces dans le traitement de la sclérodermie.
